# EUROPÄISCHE PATENTSCHRIFT

(11) **EP 0 268 227 B1**
(45) Veröffentlichungstag und Bekanntmachung des Hinweises auf die Patenterteilung: **13.01.1999**
(21) Anmeldenummer: 87116781.3
(22) Anmeldetag: 13.11.1987
(51) Int. Cl.: A61L 2/18, C11D 3/386, C11D 3/48

(54) **Verfahren zur Reinigung und Desinfektion von Endoskopen und Mittel zur Durchführung des Verfahrens**
Method for cleaning and disinfecting endoscopes, and product for carrying out the method
Procédé de nettoyage et de désinfection d'endoscopes et produit pour la mise en oeuvre du procédé

(30) Priorität: 17.11.1986 DE 3639322
(43) Veröffentlichungstag der Anmeldung: 25.05.1988
(73) Patentinhaber: Henkel Kommanditgesellschaft auf Aktien, 40191 Düsseldorf (DE)
(72) Erfinder: Disch, Karlheinz, Dr., D-5657 Haan (DE); Hachmann, Klaus, Dr., D-4010 Hilden (DE); Bansemir, Klaus, Dr., D-4018 Langenfeld (DE)

(56) Entgegenhaltungen:
- EP-A- 0 028 865
- EP-A- 0 117 183
- EP-A- 0 118 933
- EP-A- 0 158 869
- EP-A- 0 224 971
- DE-A- 3 327 466
- US-A- 3 697 222

## Beschreibung

Bei der medizinischen Diagnostik und Therapie werden chirurgische Eingriffe in steigendem Maß durch den Einsatz von Endoskopen ersetzt. Diese Entwicklung ist vor allem dadurch möglich geworden, daß seit einiger Zeit flexible Glasfiberendoskope zur Verfügung stehen. Bei der bestimmungsgemäßen Verwendung werden die Endoskope massiv mit Mikroorganismen infiziert, die sich in den Körperhöhlen, auf der Schleimhaut und im Blut befinden. Benutzte Endoskope müssen deshalb nach jedem Einsatz gründlich gereinigt und desinfiziert werden.

Glasfiberendoskope stellen außerordentlich komplizierte Präzisionsinstrumente dar, die bewegliche Teile besitzen und aus einer Vielzahl von Materialien gefertigt sind. Ihre Reinigung und Desinfektion ist aus einer Reihe von Gründen höchst problematisch. So sind jeweils nicht nur die außenliegenden Oberflächen des Instrumentes sondern auch die im Inneren vorhandenen englumigen Kanäle zu reinigen und zu desinfizieren. Mit Rücksicht auf die empfindlichen Materialien soll die Reinigung und Desinfektion so durchgeführt werden, daß auf den behandelten Oberflächen des Instrumentes keine Rückstände der verwendeten Mittel verbleiben. Die bei medizinischen Instrumenten übliche und höchst wirksame thermische Sterilisation kann hier nicht angewendet werden, da die Endoskope zum Teil aus temperaturempfindlichen Materialien gefertigt sind. Weiterhin ist zu berücksichtigen, daß eine Reihe von vorhandenen Metallteilen korrosionsanfällig ist. Schließlich soll sich die Reinigung und Desinfektion der Endoskope in kurzer Zeit durchführen lassen, damit die Instrumente möglichst schnell für die Behandlung der nächsten Patienten wieder zur Verfügung stehen. Erst vor wenigen Jahren ist es den Herstellern von Glasfiberendoskopen gelungen, Instrumente zu entwickeln, die vollständig in Reinigungs- und Desinfektionsbäder eingelegt werden können und Temperaturen von bis zu 70 °C unbeschadet aushalten

Aus der DE 33 27 466 ist ein Verfahren zum Reinigen von Gebrauchsgegenständen aus dem Bereich der Medizin und der Krankenpflege bekannt, bei dem die Gebrauchsgegenstände in einem geschlossenen System bei Temperaturen von höchstens 70 °C in einer Trägerflüssigkeit (Wasser) mit einem üblichen Reinigungsmittel behandelt werden und anschließend ein Desinfektionsmittel zusätzlich in die Trägerflüssigkeit eingegeben wird. Dabei kann als Desinfektionsmittel eine Mischung aus Glutaraldehyd und/oder Bernsteinsäuredialdehyd mit einem Salicylat und einem Polyethylenglykol eingesetzt werden. Bei der Durchführung des Verfahrens sind für die Reinigungsmittelbehandlung und die Desinfektionsmittelbehandlung separate Temperatur-Haltezeiten vorgesehen. Für die Reinigungsbehandlung werden alkalische Reinigungsmittelzusammensetzungen empfohlen. Dieses Verfahren hat sich für die Reinigung und Desinfektion von Endoskopen als nicht geeignet erwiesen, vor allem deshalb, weil es bereits nach einer begrenzten Zahl von Verfahrenszyklen zu Korrosionserscheinungen an den Metallteilen der Endoskope führt.

Die US 3 697 222 beschreibt ein Sterilisierungsverfahren für kontaminierte Gegenstände, bei dem diese bei erhöhter Temperatur mit einer wäßrigen sauren oder alkalischen Glutaraldehydlösung gegebenenfalls unter Einwirkung von Ultraschall behandelt werden. Angaben über geeignete vorhergehende Reinigungsmaßnahmen fehlen dort.

Der Erfindung lag die Aufgabe zugrunde, ein Verfahren zu entwickeln, das durch eine Kombination von thermischer und chemischer Behandlung eine zuverlässige Reinigung und Desinfektion der Endoskope in kurzer Zeit ermöglicht und auch bei Daueranwendung keine Schädigung der behandelten Instrumente zur Folge hat. Dieses Verfahren sollte so ausgelegt sein, daß es gegebenenfalls in einer automatisch arbeitenden Spülmaschine durchgeführt werden kann. Ferner sollte das Verfahren so gestaltet werden können, daß die gebrauchten Reinigungs- und Desinfektionslösungen steril sind, so daß sie ohne Bedenken dem normalen Abwasser beigemischt werden können. Diese Aufgabe wird durch das nachstehend beschriebene Verfahren gelöst.

Gegenstand der Erfindung ist ein Verfahren zur Reinigung und Desinfektion von Endoskopen mit Hilfe von wässrigen Reinigungs- und Desinfektionsmittellösungen, bei dem man die zu behandelnden Oberflächen der Endoskope nacheinander
a) mit einer Reinigungslösung in Kontakt bringt, die auf 55 bis 65 °C erhitzt, 1 bis 15 Minuten lang auf dieser Temperatur gehalten und anschließend abgetrennt wird, und die
   - mindestens ein schaumarmes nichtionogenes Tensid,
   - mindestens ein proteolytisches Enzym,
   - mindestens einen Komplexbildner und
   - gegebenenfalls weitere übliche Reinigungsmittelbestandteile
   enthält und einen pH-Wert von 6 bis 8 besitzt,
b) mit einer Desinfektionsmittellösung in Kontakt bringt, die auf 55 bis 65 °C erhitzt, 1 bis 15 Minuten auf dieser Temperatur gehalten und anschließend abgetrennt wird und die
   - mindestens einen Aldehyd aus der aus Formaldehyd und aliphatischen Dialdehyden mit 2 bis 8 Kohlenstoffatomen bestehenden Gruppe und
   - mindestens einen Komplexbildner
   enthält und einen pH-Wert im Bereich von 6 bis 8 besitzt,
c) mindestens zweimal mit Wasser spült, dessen pH-Wert auf 6 bis 8 eingestellt ist, wobei man zumindest im letzten Spülgang das Wasser auf 55 bis 65 °C erhitzt,
d) mit sterilisierter Heißluft bei 55 bis 65 °C trocknet,
   wobei man in den Schritten a) bis c) Wasser mit einer Härte von 3 bis 8 ° d) einsetzt.

Es hat sich als zweckmäßig erwiesen, im Schritt a) eine Reinigungsmittellösung einzusetzten, die
0,1 bis 1,0 g/l schaumarmes Tensid,
0,03 bis 0,3 AE/l proteolytisches Enzym und
0,03 bis 0,3 g/l Komplexbildner
enthält (AE = Anson-Einheiten).

Als schaumarme nichtionogene Tenside, die sich für die Verwendung in der Reinigungslösung des Schrittes a) eignen, sind vor allem Alkylenoxidaddukte zu nennen, wie sie durch Anlagerung von 3 bis 30 Mol Ethylenoxid und/oder Propylenoxyd an aliphatische Polyole mit 2 bis 6 Hydroxylgruppen und 2 bis 12 Kohlenstoffatomen sowie an Fettalkohole, Fettsäuren, Fettamine oder Alkylphenole mit jeweils 8 bis 18 Kohlenstoffatomen erhalten werden können, wobei die endständigen Hydroxylgruppen dieser Polyglykoletherderivate auch verethert, verestert oder acetalisiert sein können. Besonders geeignet sind Anlagerungsprodukte von 3 bis 15 Mol Ethylenoxyd an gesättigte und ungesättigte Fettalkohole mit 8 bis 18 Kohlenstoffatomen, Anlagerungsprodukte von 3 bis 5 Mol Ethylenoxid und 3 bis 6 Mol Propylenoxid an gesättigte und ungesättigte Fettalkohole mit 8 bis 18 Kohlenstoffatomen, wobei diese gemischten Alkylenoxidaddukte sowohl in Random- als auch im Blockpolymerisationsverfahren hergestellt sein können, sowie Etherderivate der vorgenannten Fettalkoholalkylenglykolether, in denen die endständigen Hydroxylgruppen mit einem geradkettigen oder verzweigten gesättigten aliphatischen Alkohol mit 4 bis 8 Kohlenstoffatomen verethert sind. Von besonderer Bedeutung sind hier Polyethylenglykolether der Formel I,

R¹ - O - (CH₂CH₂O)ₙ - R² (I),

in der R¹ einen geradkettigen oder verzweigten Alkyl- oder Alkenylrest mit 8 bis 18 Kohlenstoffatomen, R² einen geradkettigen oder verzweigten Alkylrest mit 4 bis 8 Kohlenstoffatomen und n eine Zahl von 7 bis 12 bedeutet, wobei hier Polyethylenglykolether der Formel I besonders bevorzugt sind, in der R¹ für einen aus einem gehärteten oder ungehärteten Talgfettalkohol stammendes Gemisch aus Alkyl- und/oder Alkenylresten mit 12 bis 18 Kohlenstoffatomen und R² für einen n-Butylrest steht, während n eine Zahl von 9 bis 10 bedeutet.

Als proteolytische Enzyme kommen für die Reinigungslösung des Schrittes a) insbesondere aus Bakterienstämmen gewonnene Proteasen in Betracht. Geeignet sind z.B. die aus Bacillus subtilis, Bacillus licheniformis und Streptomyces griseus gewonnenen Enzyme. Entsprechende handelsübliche Präparate liegen entweder in Form von Lösungen des Enzyms in einem Gemisch aus Wasser und einem organischen Lösungsmittel, beispielsweise 1,2-Propandiol, oder als feste Granulate vor. Diese Handelsformen enthalten in der Regel wasserlösliche Calciumsalze als Potenzierungs- und Stabilisierungsmittel. Feste Präparate sind mittels Stellmittel, beispielsweise Natriumsulfat, Natriumchlorid, Alkaliphosphat oder Alkalipolyphosphat auf einen bestimmten Aktivitätsgrad eingestellt.

Als Komplexbildner kann die Reinigungslösung des Schrittes a) beispielsweise Alkalisalze der Nitrilotriessigsäure, Ethylendiaminotetraessigsäure, 1-Hydroxyethan-1,1-diphosphonsäure, Aminotris-(methylenphosphonsäure), Ethylendiamiotetrakis-(methylenphosphonsäure), Phosphonobutantricarbonsäure, Weinsäure, Citronensäure und Glukonsäure enthalten. Besonders bevorzugt ist hier Natriumglukonat.

Die Desinfektionsmittellösung des Schrittes b) enthält zweckmäßigerweise
0,5 bis 5 g/l Aldehyd und
0,02 bis 0,25 g/l Komplexbildner.

Beispiele für die in der Desinfektionsmittellösung des Schrittes b) vorhandenen aliphatischen Dialdehyde mit 6 bis 8 Kohlenstoffatomen sind Glyoxal, Malonaldehyd, Succinaldehyd und Glutaraldehyd. Vorzugsweise enthält die in Schritt b) des erfindungsgemäßen Verfahrens eingesetzte Desinfektionsmittellösung Glutaraldehyd.

Als Komplexbildner kann die Desinfektionsmittellösung des Schrittes b) dieselben Substanzen enthalten, die weiter oben als mögliche Bestandteile der Reinigungslösung in Schritt a) genannt wurden. Vorzugsweise werden Natriumsalze der Phosphonobutantricarbonsäure in der Desinfektionsmittellösung des Schrittes b) eingesetzt.

Bei der Durchführung des erfindungsgemäßen Verfahrens wird prinzipiell Wasser mit einer Härte im Bereich von 3 bis 8 °d eingesetzt. Dies gilt sowohl für das Ansetzen der Reinigungs-und der Desinfektionsmittellösung als auch für die Spülgänge. Die Einstellung der vorgenannten Härtegrade erfolgt zweckmäßigerweise dadurch, daß man Leitungswasser zumindest anteilweise über einen Kationenaustauscher leitet, der die härtebildenden Kationen aus dem Wasser entfernt. Dieser Kationenaustausch hat eine Verschiebung des pH-Wertes in den alkalischen Bereich zur Folge. Aus diesem Grund ist es erforderlich, daß der pH-Wert jeweils auf den angegebenen Bereich von 6 bis 8 eingestellt wird. Die Reinigungslösung und die Desinfektionsmittellösung werden in der Regel aus Konzentraten hergestellt, die im Folgenden noch beschrieben werden. Derartige Konzentrate lassen sich so formulieren, daß sie beim Verdünnen mit dem kationenaustauscherbehandelten Leitungswasser Lösungen mit einem pH-Wert im geforderten Bereich ergeben. Das für die Spülgänge verwendete Wasser wird mit Hilfe von physiologisch unbedenklichen organischen Säuren, beispielsweise mit Essigsäure, Weinsäure, Milchsäure, Äpfelsäure, Citronensäure oder auf einen pH-Wert im Bereich von 6 bis 8 eingestellt.

Die gebrauchten Reinigungsmittellösungen aus dem Schritt a) werden vor dem Ablassen in die Kanalisation zweckmäßigerweise desinfiziert. Dazu kann man die Reinigungslösungen vor dem Abtrennen mit einer konzentrierten Desinfektionsmittellösung auf Basis von Aldehyden aus der aus Formaldehyd und aliphatischen Dialdehyden mit 2 bis 8 Kohlenstoffatomen bestehenden Gruppe und Komplexbildnern in einer solchen Menge versetzen, daß nach der Zugabe in der gesamten Lösung 0,25 bis 2,5 g/l Aldehyd und 0,01 bis 0,13 g/l Komplexbildner vorhanden sind.

Gewünschtenfalls kann zwischen dem Schritt a) und dem Schritt b) ein Spülgang eingeschaltet werden, der dann wiederum mit Wasser durchgeführt wird, dessen pH-Wert auf 6 bis 8 eingestellt ist.

Während der Behandlung der Endoskope mit der Reinigungs- und der Desinfektionsmittellösung in den Schritten a) und b) des erfindungsgemäßen Verfahrens können die Endoskope gleichzeitig -zur Verstärkung der reinigenden bzw. der desinfizierenden Wirkung - der Einwirkung von Ultraschall ausgesetzt werden.

Zum Trocknen der Endoskope im Schritt d) verwendet man vorzugsweise Luft, die vor dem Erhitzen durch Ansaugen über einen Mikrofilter sterilisiert wurde.

Die praktische Durchführung des erfindungsgemäßen Verfahrens kann beispielsweise in entsprechend dimensionierten, verschließbaren Edelstahlbehältern erfolgen, die beheizbar sind und mit einer Einrichtung zum Umpumpen der verschiedenen Flüssigkeiten und der zur Trocknung verwendeten Heißluft durch die zu reinigenden Kanäle der Endoskope versehen sind. Weiterhin sollen Zu- und Ableitungen für die Reinigungs- und die Desinfektionslösung und das Spülwasser sowie für die zur Trocknung der Instrumente benötigte Heißluft vorhanden sein. Es ist von Vorteil, wenn die zu behandelnden Endoskope in ein in den Edelstahlbehälter passendes Gestell eingelegt werden können. Für die Durchführung der einzelnen Schritte des erfindungsgemäßen Verfahrens wird der Behälter jeweils mit so viel Flüssigkeit beschickt, daß die Endoskope vollständig damit bedeckt sind. Die jeweils vorhandene Flüssigkeit wird ständig mit einer ausreichenden Geschwindigkeit durch die Kanäle des Endoskops umgepumpt. Beim Ablassen der Behandlungsflüssigkeiten ist dafür Sorge zu tragen, daß auch die jeweils in den Kanälen vorhandene Flüssigkeit entfernt wird.

Für die Durchführung des erfindungsgemäßen Verfahrens eignen sich insbesondere automatisch arbeitende Spülmaschinen, wie sie für die Reinigung von Laborgeräten und medizinischen Instrumenten bekannt und üblich sind, unter der Voraussetzung, daß sie die erforderlichen Zusatzeinrichtungen aufweisen, beispielsweise eine Einrichtung, mit deren Hilfe die Flüssigkeiten durch die Kanäle der Endoskope bewegt werden können. Die äußeren Flächen der Endoskope werden hier nicht durch Einlegen in die Flüssigkeiten mit diesen in Kontakt gebracht, sondern durch laufendes Besprühen.

Für die Herstellung der Reinigungs- und Desinfektionsmittellösungen werden in der Regel beständige lagerfähige Konzentrate bereitgestellt, die außer den im Vorstehenden bereits genannten Wirkstoffen noch weitere in solchen Konzentraten üblichen Bestandteile enthalten.

Ein wäßriges Reinigungsmittelkonzentrat für die Herstellung der in Schritt a) verwendeten Reinigungslösung kann beispielsweise
5 bis 10 Gew.-% schaumarmes nichtionogenes Tensid,
7,1 bis 77 AE/l proteolytisches Enzym,
1 bis 5 Gew.-% Komplexbildner,
10 bis 50 Gew.-% Enzymstabilisator,
1 bis 5 Gew.-% Konfektionierhilfsmittel und
0,05 bis 0,5 Gew.-% Konservierungsmittel
enthalten. Der pH-Wert des Konzentrates wird mit Säure, Base oder einem Säure-Basengemisch auf 4 bis 6 eingestellt.

Als Enzymstabilisator kann das wässrige Reinigungsmittelkonzentrat beispielsweise Triethanolamin, Morpholin, alpha-Pyrrolidon, Ethylenglykol, Propylenglykol, Glycerin, wasserlösliche Calciumsalze oder Gemische dieser Verbindungen enthalten. Vorzugsweise wird hier Glycerin und/oder Propylenglykol als Enzymstabilisator eingesetzt.

Für das wässrige Reinigungsmittelkonzentrat geeignete Konfektionierhilfsmittel (Lösungsvermittler) sind beispielsweise Natriumcumolsulfonat, Natriumtoluolsulfonat, Natriumxylolsulfonat, Harnstoff, Polyethylenglykole, Methylacetamid und Fettalkohole, wie Cetylalkohol, bevorzugt wird Natriumcumolsulfonat als Konfektionierhilfsmittel eingesetzt.

Reinigungsmittelkonzentrate der beschriebenen Art sind gegen Mikrobenbefall anfällig. Insbesondere wird bei nichtkonservierten Zusammensetzungen leicht Pilzwachstum beobachtet. Aus diesem Grund setzt man den Konzentraten wirksame Mengen Konservierungsmittel zu. Geeignete Konservierungsmittel sind beispielsweise p-Hydroxybenzoesäuremethylester, 5-Brom-5-nitro-1,3-dioxan, Glutaraldehyd, Salicylsäure, 0-2-Naphthyl-m-N-dimethylthiocarbanilat, 5-Chlor-5-methyl-4-isothiazolin-3-on, 2-Methyl-4-isothiazolin-3-on und Gemische der beiden zuletzt genannten Verbindungen. Vorzugsweise wird p-Hydroxybenzoesäuremethylester als Konservierungsmittel eingesetzt.

Für die in dem wässrigen Reinigungsmittelkonzentrat enthaltenen Bestandteile schaumarmes nichtionogenes Tensid, proteolytisches Enzym und Komplexbildner gelten die in der vorstehenden Beschreibung des erfindungsgemäßen Verfahrens enthaltenen Angaben in vollem Umfang.

Ein wässriges Desinfektionsmittelkonzentrat für die Herstellung der in Schritt b) verwendeten Desinfektionsmittellösung kann beispielsweise
10 bis 40 Gew.-% Aldehyd aus der aus Formaldehyd und aliphatischen Dialdehyden mit 2 bis 8 Kohlenstoffatomen bestehenden Gruppe
0,5 bis 2 Gew.-% Komplexbildner und
7 bis 15 Gew.-% Konfektionierhilfsmittel
enthalten. Der pH-Wert des Konzentrates wird mit Säure, Base oder einem Säure-Basengemisch auf 3 bis 5 eingestellt.

Als Konfektionierhilfsmittel für das Desinfektionsmittelkonzentrat kommen insbesondere niedere aliphatische Alkohole wie Ethanol, n-Propanol und Isopropanol sowie Ethylenglykol und Triacetin in Betracht. Vorzugsweise wird Ethanol als Konfektionierhilfsmittel verwendet.

Bezüglich derin dem wässrigen Desinfektionsmittelkonzentrat enthaltenen Bestandteile aliphatischer Dialdehyd und Komplexbildner gelten wiederum die in der vorstehenden Beschreibung des erfindungsgemäßen Verfahrens enthaltenen Angaben in vollem Umfang.

### Beispiel

Durch mechanisches Vereinigen der Einzelbestandteile wurden Konzentrate der folgenden Zusammensetzung (GT = Gewichtsteile) hergestellt:

### Reinigungsmittelkonzentrat

8 GT n-Butylether eines Anlagerungsproduktes von
9,5 Mol Ethylenoxid an 1 Mol gehärteten Talgfettsalkohol (Formel I: R¹ = C8₁₂₋₁₈-Alkyl; R² = C₄-Alkyl; n = 9,5)
1 GT proteolytisches Enzym (Alcalase ^{R}, Hersteller: Novo Industri A/S, Bagsvaerd, Dänemark; 2,5 AE/g)
6 GT Glycerin
3 GT 1,2-Propylenglykol
2,5 GT Natriumgluconat
2 Gt Citronensäure
3 GT Natriumcumolsulfonat
0,1 GT p-Hydroxybenzoesäuremethylester
ad 100 GT Wasser

Das Gemisch wurde mit 37 gew.-%iger Natriumhydroxydlösung auf pH 5 eingestellt.

### Desinfektionsmittelkonzentrat

20 GT Glutaraldehyd
1 GT Phosphonobutantricarbonsäure
8 GT Ethanol
ad 100 GT Wasser

Das Gemisch wurde mit 50 gew.-%iger Natriumhydroxydlösung auf pH 4 eingestellt.

Die Reinigung und Desinfektion der Endoskope erfolgte in einem verschließbaren, mit einer Heizung versehenen Edelstahlgefäß (Durchmesser ca. 60 cm; Höhe ca. 65 cm), das Zu- und Ableitungen für die Reinigungs- und Desinfektionsmittellösung und das in den Spülgängen benutzte Wasser sowie für die zur Trocknung der Instrumente benötigte Heißluft besaß. Die Apparatur war mit einer Umlaufpumpe versehen, mit deren Hilfe die jeweils vorhandene Flüssigkeit durch die Kanäle der Fiberendoskope gepumpt werden konnte.

Die Versuche wurden mit einem handelsüblichen Gastroskop durchgeführt.

Zum Ansetzen der Reinigungs- und der Desinfektionsmittellösung wurde Wasser verwendet, das mit Hilfe eines Kationenaustauschers auf eine Härte von 5° d eingestellt war. Dasselbe Wasser wurde zur Durchführung der Spülgänge eingesetzt, nachdem es mit Hilfe von Milchsäure auf pH 7 eingestellt war.

Durch Verdünnen des Reinigungsmittelkonzentrates wurde eine Reinigungslösung hergestellt, die 0,45 g/l Tensid, 0,06 g/l Enzym und 0,14 g/l Natriumglukonat enthielt. Durch Verdünnen des Desinfektionsmittelkonzentrates wurde eine anwendungsfähige Desinfektionsmittellösung hergestellt, die 2,4 g/l Glutaraldehyd und 0,12 g/l Phosphonobutantricarbonsäure enthielt.

Die für die Trocknung eingesetzte Luft wurde über ein Mikrofilter angesaugt und vor dem Einleiten in das Edelstahlgefäß durch eine Heizstrecke geleitet, wo sie auf 60 °C erwärmt wurde.

Bei der Durchführung des Reinigungsverfahrens wurde das Endoskop in einem Drahtkorb in den Edelstahlbehälter eingelegt. Die Kanäle des Endoskops wurden an die Umlaufpumpe angeschlossen. In den einzelnen Schritten des Verfahrens wurde dem Edelstahlbehälter jeweils soviel Flüssigkeit zugeführt, daß das Endoskop ganz bedeckt war. Während der einzelnen Verfahrensschritte wurde die vorhandene Flüssigkeit laufend durch die Kanäle des Endoskops umgepumpt.

Nach dem Befüllen des Edelstahlgefäßes mit Reinigungslösung wurde diese auf 60 °C erhitzt und 10 Minuten lang auf dieser Temperatur gehalten. Anschließend wurde die Reinigungslösung abgelassen und durch die Desinfektionsmittellösung ersetzt, die wiederum auf 60 °C erhitzt und 10 Minuten lang auf dieser Temperatur gehalten wurde. Nach dem Abtrennen der Desinfektionsmittellösung wurde zweimal mit kaltem Wasser gespült. Anschließend wurde das Edelstahlgefäß erneut mit Wasser gefüllt, das nun auf 60 °C erhitzt und danach abgezogen wurde. Zur Trocknung des Endoskops wurde schließlich 5 Minuten lang sterile Heißluft eingeleitet.

In einer Abwandlung des Verfahrens wurde die Reinigungslösung des Schrittes a) vor dem Abtrennen mit soviel Desinfektionsmittelkonzentrat versetzt, daß in der gesamten Lösung 1,2 g/l Glutaraldehyd und 0,06 g/l Phosphonobutantricarbonsäure vorhanden waren.

Zur Prüfung der bei dem erfindungsgemäßen Verfahren erzielten desinfizierenden Wirkung wurden die Kanäle des Endoskops mit einer Keimsuspension kontaminiert, die in einer ersten Versuchsserie ein Gemisch folgender Keime enthielt:
1) ca. 10⁸ Keime/ml Staphylococcus aureus
2) ca. 10⁸ Keime/ml Escherichia coli
3) ca. 10⁸ Keime/ml Pseudomonas aeroginosa
4) ca. 10⁸ Keime/ml Proteus mirabilis
5) ca. 10⁸ Keime/ml Candida albicans

In einer zweiten Versuchsserie enthielt die Keimdispersion nur
6) ca. 10⁸ Keime/ml Streptococcus faecalis.

Zur Simulierung praxisnaher Bedingungen enthielten die Keimsuspensionen einen Zusatz von 20 Gewichtsprozent defibrinierten Hammelblutes.

Bei der Kontaminierung wurden die Kanäle des Endoskops mit den Keimsuspensionen gefüllt. Nach kurzem Stehenlassen wurden die Keimsuspensionen wieder abgelassen. Jeweils eine Stunde nach der Kontamination wurde das Endoskop erfindungsgemäß gereinigt und desinfiziert. Anschließend wurden 0,5 l einer Lösung, die 3 Gewichtsprozent Tween 80, 0,3 Gewichtsprozent Lecithin, 0,1 Gewichtsprozent Histidin, 0,1 Gewichtsprozent Trypton und 0,05 Gewichtsprozent Natriumchlorid enthielt, durch die Kanäle des Endoskops gesaugt. Proben von jeweils 1 ml dieser Lösung wurden auf Aggarplatten überimpft, die anschließend mindestens 48 Stunden bei 37 °C beziehungsweise mindestens 72 Stunden bei 35 °C bebrütet und danach auf vorhandenes Keimwachstum geprüft wurden.

Es wurde festgestellt, daß bei der Durchführung des erfindungsgemäßen Verfahrens in allen Fällen die geforderte Keimfreiheit erzielt worden war.

## Patentansprüche

1. Verfahren zur Reinigung und Desinfektion von Endoskopen, bei dem man die zu behandelnden Oberflächen der Endoskope nacheinander a) mit einer Reinigungslösung in Kontakt bringt, die mindestens ein schaumarmes nichtionogenes Tensid, mindestens ein proteolytisches Enzym, mindestens einen Komplexbildner und gegebenenfalls weitere übliche Reinigungsmittelbestandteile enthält, b) mit einer Desinfektionsmittellösung in Kontakt bringt, die mindestens einen Aldehyd aus der Formaldehyd und aliphatische Dialdehyde mit 2 bis 8 Kohlenstoffatomen bestehenden Gruppe und mindestens einen Komplexbildner enthält und c) mindestens zweimal mit Wasser spült, dadurch gekennzeichnet, daß in Stufe a) die Reinigungslösung einen pH-Wert von 6 bis 8 besitzt, auf 55 bis 65 °C erhitzt wird, 1 bis 15 Minuten bei dieser Temperatur gehalten und anschließend abgetrennt wird, daß in Stufe b) die Desinfektionsmittellösung einen pH-Wert von 6 bis 8 besitzt, auf 55 bis 65 °C erhitzt wird und 1 bis 15 Minuten bei dieser Temperatur gehalten wird, daß in Stufe c) das Spülwasser auf einen pH-Wert von 6 bis 8 eingestellt ist, wobei man zumindest im letzten Spülgang das Wasser auf 55 bis 65 °C erhitzt und daß man in einer Stufe d) nach dem Spülen mit sterilisierter Heißluft bei 55 bis 65 °C trocknet, wobei man in den Schritten a) bis c) Wasser mit einer Härte von 3 bis 8 °dH einsetzt.

2. Verfahren nach Anspruch 1, dadurch gekennzeichnet, daß die Reinigungslösung im Schritt a)
0,1 bis 1,0 g/l schaumarmes Tensid,
0,03 bis 0,3 AE/l proteolytisches Enzym und
0,03 bis 0,3 g/l Komplexbildner
enthält (AE = Anson-Eiheiten).

3. Verfahren nach den Ansprüchen 1 und 2, dadurch gekennzeichnet, daß die Reinigungslösung in Schritt a) als schaumarmes nichtionogenes Tensid ein Alkylenoxidaddukt enthält, das durch Anlagerung von 3 bis 30 Mol Ethylenoxid und/oder Propylenoxid an aliphatische Polyole mit 2 bis 6 Hydroxylgruppen und 2 bis 12 Kohlenstoffatome sowie an Fettalkohole, Fettsäuren, Fettamine oder Alkylphenole mit jeweils 8 bis 18 Kohlenstoffatomen erhältlich ist, wobei die endständigen Hydroxylgruppen dieser Polyglykoletherderivate auch verethert, verestert oder acetalisiert sein können.

4. Verfahren nach den Ansprüchen 1 bis 3, dadurch gekennzeichnet, daß die Reinigungslösung in Schritt a) ein schaumarmes nichtionogenes Tensid aus der Gruppe Anlagerungsprodukte von 3 bis 15 Mol Ethylenoxid an gesättigte und ungesättigte Fettalkohole mit 8 bis 18 Kohlenstoffatomen, Anlagerungsprodukte von 3 bis 5 Mol Ethylenoxid und 3 bis 6 Mol Propylenoxid an gesättigte und ungesättigte Fettalkohole mit 8 bis 18 Kohlenstoffatomen und Etherderivate dieser Fettalkoholpolyalkylenglykolether, in denen die endständigen Hydroxylgruppen mit einem geradkettigen oder verzweigten gesättigten aliphatischen Alkohol mit 4 bis 8 Kohlenstoffatomen verethert sind, enthält.

5. Verfahren nach Anspruch 4, dadurch gekennzeichnet, daß die Reinigungslösung in Schritt a) als schaumarmes Tensid einen Polyethylenglykolether der Formel I enthält,
R¹ - O - (CH₂CH₂O)ₙ - R² (I),
in der R¹ einen geradkettigen oder verzweigten Alkyl- oder Alkenylrest mit 8 bis 18 Kohlenstoffatomen, R² einen geradkettigen oder verzweigten Alkylrest mit 4 bis 8 Kohlenstoffatomen und n eine Zahl von 7 bis 12 bedeutet.

6. Verfahren nach Anspruch 5, dadurch gekennzeichnet, daß die Reinigungslösung in Schritt a) einen Polyethylenglykolether der Formel I in Anspruch 5 enthält, in der R¹ für ein aus einem Talgfettalkohol stammendes Gemisch aus Alkyl- und/oder Alkenylresten mit 12 bis 18 Kohlenstoffatomen und R² für einen n-Butylrest steht, während n eine Zahl von 9 bis 10 bedeutet.

7. Verfahren nach den Ansprüchen 1 bis 6, dadurch gekennzeichnet, daß die Reinigungslösung in Schritt a) einen Komplexbildner aus der Gruppe Alkalisalze der Nitrilotriessigsäure, Ethylendiaminotetraessigsäure, 1-Hydroxyethan-1,1-diphosphonsäure, Aminotris-(methylenphosphonsäure), Ethylendiaminotetrakis-(methylenphosphonsäure), Phosphonobutantricarbonsäure, Weinsäure, Citronensäure und Glukonsäure enthält.

8. Verfahren nach Anspruch 7, dadurch gekennzeichnet, daß die Reinigungslösung in Schritt a) als Komplexbildner Natriumglukonat enthält.

9. Verfahren nach den Ansprüchen 1 bis 8, dadurch gekennzeichnet, daß die Desinfektionsmittellösung in Schritt b)
0,5 bis 5 g/l Aldehyd und
0,02 bis 0,25 g/l Komplexbildner
enthält.

10. Verfahren nach den Ansprüchen 1 bis 9, dadurch gekennzeichnet, daß die Desinfektionsmittellösung in Schritt b) Glutaraldehyd enthält.

11. Verfahren nach den Ansprüchen 1 bis 10, dadurch gekennzeichnet, daß die Desinfektionsmittellösung in Schritt b) einen Komplexbildner aus der Gruppe Alkalisalze der Nitrilotriessigsäure, Ethylendiaminotetraessigsäure, 1-Hydroxyethan-1,1-diphosphonsäure, Aminotris-(methylenphosphonsäure), Ethylendiaminotetrakis-(methylenphosphonsäure), Phosphonobutantricarbonsäure, Weinsäure, Citronensäure und Glukonsäure enthält.

12. Verfahren nach Anspruch 11, dadurch gekennzeichnet dadurch gekennzeichnet, daß die Desinfektionsmittellösung in Schritt b) als Komplexbildner ein Natriumsalz der Phosphonobutantricarbonsäure enthält.

13. Verfahren nach den Ansprüchen 1 bis 12, dadurch gekennzeichnet, dadurch gekennzeichnet, daß im Schritt a) die Reinigungslösung vor dem Abtrennen mit einer Desinfektionsmittellösung, die mindestens einen Aldehyd aus der aus Formaldehyd mit 2 bis 8 Kohlenstoffatomen bestehenden Gruppe und mindestens einen Komplexbildner enthält, versetzt wird.

14. Verfahren nach Anspruch 13, dadurch gekennzeichnet, daß die Reinigungslösung mit so viel Desinfektionsmittellösung versetzt wird, daß in der gesamten Lösung
0,25 bis 2,5 g/l Aldehyd und
0,01 bis 0,13 g/l Komplexbildner
vorhanden sind.

15. Verfahren nach den Ansprüchen 1 bis 14, dadurch gekennzeichnet, daß man die zu behandelnden Endoskope zwischen dem Schritt a) und dem Schritt b) mindestens einmal mit Wasser spült, dessen pH-Wert auf 6 bis 8 eingestellt ist.

16. Verfahren nach den Ansprüchen 1 bis 15, dadurch gekennzeichnet, daß man im Schritt a) und/oder im Schritt b) die Endoskope der Einwirkung von Ultraschall aussetzt.

17. Verfahren nach den Ansprüchen 1 bis 16, dadurch gekennzeichnet, daß man die zu behandelnden Endoskope im Schritt d) mit Heißluft behandelt, die mit Hilfe eines Mikrofilters sterilisiert wurde.

18. Wäßriges Reinigungsmittelkonzentrat für die Durchführung des Verfahrens nach einem der Ansprüche 1 bis 17, enthaltend 5 bis 10 Gew.-% eines schaumarmen nichtionogenen Tensids, proteolytisches Enzym, Komplexbildner, Enzymstabilisator, Konfektionierungshilfsmittel und Konservierungsmittel, dadurch gekennzeichnet, daß das schaumarme nichtionogene Tensid die Formel
R¹-O-(CH₂-CH₂O)ₙ-R² (I)
aufweist, in der R¹ einen geradkettigen oder verzweigten Alkyl- oder Alkenylrest mit 8 bis 18 Kohlenstoffatomen R² einen geradkettigen oder verzweigten Alkylrest mit 4 bis 8 Kohlenstoffatomen und n eine ganze Zahl von 7 bis 12 bedeutet, der Gehalt an proteolytischem Enzym in der Lösung 7,7 bis 77 AE/l, der Gehalt an Komplexbildner 1 bis 5 Gew.-%, der Gehalt an Enzymstabilisator 10 bis 50 Gew.-%, der Gehalt an Konfektionierungshilfsmittel 1 bis 5 Gew.-% und der Gehalt an Konservierungsmittel 0,05 bis 0,5 Gew.-% beträgt und dessen pH-Wert auf 4 bis 6 eingestellt ist.

19. Wässriges Reinigungsmittelkonzentrat nach Anspruch 18, dadurch gekennzeichnet, daß es als Tensid einen Polyeethylenglykolether der Formel I enthält, in der R¹ für ein aus einem Talgfettalkohol stammendes Gemisch aus Alkyl- und/oder Alkenylresten mit 12 bis 18 Kohlenstoffatomen und R² für einen n-Butylrest steht, während n eine Zahl von 9 bis 10 bedeutet.

20. Wässriges Reinigungsmittelkonzentrat nach den Ansprüchen 18 bis 19, dadurch gekennzeichnet, daß es einen Komplexbildner aus der Gruppe Alkalisalze der Nitrilotriessigsäure, Ethylendiamintetraessigsäure, 1-Hydroxyethan-1,1-diphosphonsäure, Aminotris-(methylenphosphonsäure), Ethylendiaminotetrakis-(methylenphosphonsäure), Phosphonobutantricarbonsäure, Weinsäure, Citronensäure und Glukonsäure enthält.

21. Wässriges Reinigungsmittelkonzentrat nach Anspruch 20, dadurch gekennzeichnet, daß es als Komplexbildner Natriumglukonat enthält.

22. Wässriges Reinigungsmittelkonzentrat nach den Ansprüchen 18 bis 20, dadurch gekennzeichnet, daß es als Enzymstabilisator Triethanolamin, Morpholin, alpha-Pyrrolidon, Ethylenglykol, Propylenglykol, Glycerin, wasserlösliche Calciumsalze oder Gemische dieser Verbindungen enthält.

23. Wässriges Reinigungsmittelkonzentrat nach Anspruch 22, dadurch gekennzeichnet, daß es als Enzymstabilisator Glycerin und/oder Propylenglykol enthält.

24. Wässriges Reinigungsmittelkonzentrat nach den Ansprüchen 18 bis 23, dadurch gekennzeichnet, daß es als Konfektionierhilfsmittel Natriumcumolsulfonat, Natriumtoluolsulfonat, Natriumxylolsulfonat, Harnstoff, Polyethylenglykole, Polypropylenglykole, Methylacetamid oder Fettalkohole enthält.

25. Wässriges Reinigungsmittelkonzentrat nach Anspruch 24, dadurch gekennzeichnet, daß es als Konfektionierhilfsmittel Natriumcumolsulfonat enthält.

26. Wässriges Reinigungsmittelkonzentrat nach den Ansprüchen 18 bis 25, dadurch gekennzeichnet, daß es als Konservierungsmittel p-Hydroxybenzoesäuremethylester, 5-Brom-5-nitro-1,3-dioxan, Glutaraldehyd, Salicylsäure, 0-2-Naphthyl-m-N-dimethylthiocarbanilat, 5-Chlor-5-methyl-4-isothiazolin-3-on, 2-Methyl-4-isothiazolin-3-on oder ein Gemisch aus 5-Chlor-5-methyl-4-isothiazolin-3-on und 2-Methyl-4-isothiazolin-3-on enthält.

27. Wässriges Reinigungsmittelkonzentrat nach Anspruch 26, dadurch gekennzeichnet, daß es als Konservierungsmittel p-Hydroxybenzoesäuremethylester enthält.

28. Wäßriges Desinfektionsmittelkonzentrat zur Durchführung des Verfahrens nach einem der Ansprüche 1 bis 17, enthaltend Aldehyd aus der aus Formaldehyd und aliphatischen Dialdehyden mit 2 bis 8 Kohlenstoffatomen bestehenden Gruppe, Komplexbildner und Konfektionierungshilfsmittel, dadurch gekennzeichnet, daß der Gehalt an diesem Aldehyd 10 bis 40 Gew.-%, der Gehalt an Komplexbildner 0,5 bis 2 Gew.-% und der Gehalt an Konfektionierungshilfsmittel 7 bis 15 Gew.-% beträgt, wobei als Komplexbildner Phosphonobutantricarbonsäure enthalten ist und der pH-Wert des Mittels auf 3 bis 5 eingestellt ist.

29. Wässriges Desinfektionsmittelkonzentrat nach Anspruch 28, dadurch gekennzeichnet, daß es als aliphatischen Dialdehyd Glutaraldehyd enthält.

30. Wässriges Desinfektionsmittelkonzentrat nach den Ansprüchen 28 und 29, dadurch gekennzeichnet, daß es einen Komplexbildner aus der Gruppe Alkalisalze der Nitrilotriessigsäure, Ethylendiamintetraessigsäure, 1-Hydroxyethan-1,1-diphosphonsäure, Aminotris-(methylenphosphonsäure), Ethylendiaminotetratris-(methylenphosphonsäure), Phosphonobutantricarbonsäure, Weinsäure, Citronensäure und Glukonsäure enthält.

31. Wässriges Desinfektionsmittelkonzentrat nach den Ansprüchen 28 bis 30, dadurch gekennzeichnet, daß es ein Konfektionierhilfsmittel aus der Gruppe Ethanol, n-Propanol, Isopropanol, Ethylenglykol und Triacetin enthält.

32. Wässriges Desinfektionsmittelkonzentrat nach Anspruch 31, dadurch gekennzeichnet, daß es als Konfektionierhilfsmittel Ethanol enthält.

## Claims

1. A process for cleaning and disinfecting endoscopes in which the endoscope surfaces to be treated are successively
a) brought into contact with a cleaning solution containing at least one low-foam nonionic surfactant, at least one proteolytic enzyme, at least one complexing agent and optionally other standard detergent ingredients,
b) brought into contact with a disinfectant solution containing at least one aldehyde from the group consisting of formaldehyde and aliphatic C₂₋₈ dialdehydes and at least one complexing agent and
c) rinsed at least twice with water,
characterized in that in step a) the cleaning solution has a pH valueof 6 to 8, is heated to 55 to 65°C, is kept at that temperature for 1 to 15 minutes and is then removed, in step b) the disinfectant solution has a pH value of 6 to 8, is heated to 55 to 65°C and is kept at that temperature for 1 to 15 minutes, in step c) the rinsing water is adjusted to a pH value of 6 to 8, the water being heated to 55 to 65°C at least in the last wash cycle, and in that in a step d) carried out after rinsing the endoscope surfaces are dried with sterilized hot air at 55 to 65°C, water having a hardness of 3 to 8°dH (German hardness) being used in steps a) to c).

2. A process as claimed in claim 1, characterized in that the cleaning solution in step a) contains
0.1 to 1.0 g/l of low-foam surfactant,
0.03 to 0.3 of AU/l proteolytic enzyme and
0.03 to 0.3 g/l of complexing agent
(AU = Anson Units).

3. A process as claimed in claims 1 and 2, characterized in that the cleaning solution in step a) contains as the low-foam nonionic surfactant an alkylene oxide adduct of the type obtainable by addition of 3 to 30 moles of ethylene oxide and/or propylene oxide to aliphatic polyols containing 2 to 6 hydroxyl groups and 2 to 12 carbon atoms and to fatty alcohols, fatty acids, fatty amines or alkylphenols each containing 8 to 18 carbon atoms, the terminal hydroxyl groups of these polyglycol ether derivatives optionally being etherified, esterified or acetalized.

4. A process as claimed in claims 1 to 3, characterized in that the cleaning solution in step a) contains a low-foam nonionic surfactant from the group consisting of adducts of 3 to 15 moles of ethylene oxide with saturated and unsaturated C₈₋₁₈ fatty alcohols, adducts of 3 to 5 moles of ethylene oxide and 3 to 6 moles of propylene oxide with saturated and unsaturated C₈₋₁₈ fatty alcohols and ether derivatives of these fatty alcohol polyalkylene glycol ethers in which the terminal hydroxyl groups are etherified with a straight-chain or branched-chain saturated aliphatic C₄₋₈ alcohol.

5. A process as claimed in claim 4, characterized in that the cleaning solution in step a) contains as low-foam surfactant a polyethylene glycol ether corresponding to the following formula:
R¹ - O - (CH₂CH₂O)ₙ - R² (I)
in which R¹ is a straight-chain or branched-chain C₈₋₁₈ alkyl or alkenyl radical, R² is a straight-chain or branched-chain C₄₋₈ alkyl radical and n is a number of 7 to 12.

6. A process as claimed in claim 5, characterized in that the cleaning solution in step a) contains a polyethylene glycol ether corresponding to formula I in claim 5 in which R¹ is a mixture of C₁₂₋₁₈ alkyl and/or alkenyl radicals emanating from a tallow fatty alcohol and R² is an n-butyl radical while n is a number of 9 to 10.

7. A process as claimed in claims 1 to 6, characterized in that the cleaning solution in step a) contains a complexing agent from the group consisting of alkali metal salts of nitrilotriacetic acid, ethylenediamine tetraacetic acid, 1-hydroxyethane-1,1-diphosphonic acid, aminotris-(methylenephosphonic acid), ethylenediamine tetrakis-(methylenephosphonic acid), phosphonobutane tricarboxylic acid, tartaric acid, citric acid and gluconic acid.

8. A process as claimed in claim 7, characterized in that the cleaning solution in step a) contains sodium gluconate as complexing agent.

9. A process as claimed in claims 1 to 8, characterized in that the disinfectant solution in step b) contains
0.5 to 5 g/l of aldehyde and
0.02 to 0.25 g/l of complexing agent.

10. A process as claimed in claims 1 to 9, characterized in that the disinfectant solution in step b) contains glutaraldehyde.

11. A process as claimed in claims 1 to 10, characterized in that the disinfectant solution in step b) contains a complexing agent from the group consisting of alkali metal salts of nitrilotriacetic acid, ethylenediamine tetraacetic acid, 1-hydroxyethane-1,1-diphosphonic acid, amino-tris-(methylenephosphonic acid), ethylenediamine tetrakis-(methylenephosphonic acid), phosphonobutane tricarboxylic acid, tartaric acid, citric acid and gluconic acid.

12. A process as claimed in claim 11, characterized in that the disinfectant solution in step b) contains a sodium salt of phosphonobutane tricarboxylic acid as complexing agent.

13. A process as claimed in claims 1 to 12, characterized in that, in step a), a disinfectant solution containing at least one aldehyde from the group consisting of C₂₋₈ formaldehyde and at least one complexing agent is added to the cleaning solution before drainage.

14. A process as claimed in claim 13, characterized in that disinfectant solution is added to the cleaning solution in such a quantity that the solution as a whole contains
0.25 to 2.5 g/l of aldehyde and
0.01 to 0.13 g/l of complexing agent.

15. A process as claimed in claims 1 to 14, characterized in that, between step a) and step b), the endoscopes to be treated are washed at least once with water adjusted to pH 6-8.

16. A process as claimed in claims 1 to 15, characterized in that the endoscopes are ultrasonicated in step a) and/or step b).

17. A process as claimed in claims 1 to 16, characterized in that, in step d), the endoscopes to be treated are treated with hot air sterilized by means of a microfilter.

18. An aqueous detergent concentrate for carrying out the process claimed in any of claims 1 to 17 containing 5 to 10% by weight of a low-foam nonionic surfactant, proteolytic enzyme, complexing agent, enzyme stabilizer, formulation aids and preservatives, characterized in that the low-foam nonionic surfactant corresponds to the following formula:
R¹ - O - (CH₂CH₂O)ₙ - R² (I)
in which R¹ is a straight-chain or branched-chain C₈₋₁₈ alkyl or alkenyl radical, R² is a straight-chain or branched-chain C₄₋₈ alkyl radical and n is a number of 7 to 12, the content of proteolytic enzyme in the solution is 7.7 to 77 AU/l, the content of complexing agent is 1 to 5% by weight, the content of enzyme stabilizer is 10 to 50% by weight, the content of formulation aid is 1 to 5% by weight and the content of preservative is 0.05 to 0.5% by weight and the pH is adjusted to a value of 4 to 6.

19. An aqueous detergent concentrate as claimed in claim 18, characterized in that it contains as surfactant a polyethylene glycol ether corresponding to formula I in which R¹ is a mixture of C₁₂₋₁₈ alkyl and/or alkenyl radicals emanating from a tallow fatty alcohol and R² is an n-butyl radical while n is a number of 9 to 10.

20. An aqueous detergent concentrate as claimed in claims 18 and 19, characterized in that it contains a complexing agent from the group consisting of alkali metal salts of nitrilotriacetic acid, ethylenediamine tetraacetic acid, 1-hydroxyethane-1,1-diphosphonic acid, amino-tris-(methylenephosphonic acid), ethylenediamine tetrakis-(methylenephosphonic acid), phosphonobutane tricarboxylic acid, tartaric acid, citric acid and gluconic acid.

21. An aqueous detergent concentrate as claimed in claim 20, characterized in that it contains sodium gluconate as complexing agent.

22. An aqueous detergent concentrate as claimed in claims 18 to 20, characterized in that it contains triethanolamine, morpholine, α-pyrrolidone, ethylene glycol, propylene glycol, glycerol, water-soluble calcium salts or mixtures of these compounds as enzyme stabilizer.

23. An aqueous detergent concentrate as claimed in claim 22, characterized in that it contains glycerol and/or propylene glycol as enzyme stabilizer.

24. An aqueous detergent concentrate as claimed in claims 18 to 23, characterized in that it contains sodium cumene sulfonate, sodium toluene sulfonate, sodium xylene sulfonate, urea, polyethylene glycols, polypropylene glycols, methyl acetamide or fatty alcohols as formulation aids.

25. An aqueous detergent concentrate as claimed in claim 24, characterized in that it contains sodium cumene sulfonate as formulation aid.

26. An aqueous detergent concentrate as claimed in claims 18 to 25, characterized in that it contains p-hydroxybenzoic acid methyl ester, 5-bromo-5-nitro-1,3-dioxane, glutaraldehyde, salicyclic acid, 0-2-naphthyl-m-N-dimethyl thiocarbanilate, 5-chloro-5-methyl-4-isothiazolin-3-one, 2-methyl-4-isothiazolin-3-one or a mixture of 5-chloro-5-methyl-4-isothiazolin-3-one and 2-methyl-4-isothiazolin-3-one as preservative.

27. An aqueous detergent concentrate as claimed in claim 26, characterized in that it contains p-hydroxybenzoic acid methyl ester as preservative.

28. An aqueous disinfectant concentrate for carrying out the process claimed in any of claims 1 to 17 containing aldehyde from the group consisting of formaldehyde and aliphatic C₂₋₈ dialdehydes, complexing agents and formulation aids, characterized in that the aldehyde content is 10 to 40% by weight, the content of complexing agent is 0.5 to 2% by weight and the content of formulation aid is 7 to 15% by weight, the complexing agent being phosphonobutane tricarboxylic acid and the pH of the concentrate being adjusted to a value of 3 to 5.

29. An aqueous disinfectant concentrate as claimed in claim 28, characterized in that it contains glutaraldehyde as the aliphatic dialdehyde.

30. An aqueous disinfectant concentrate as claimed in claims 28 and 29, characterized in that it contains a complexing agent from the group consisting of alkali metal salts of nitrilotriacetic acid, ethylenediamine tetraacetic acid, 1-hydroxyethane-1,1-diphosphonic acid, amino-tris-(methylenephosphonic acid), ethylenediamine tetrakis-(methylenephosphonic acid), phosphonobutane tricarboxylic acid, tartaric acid, citric acid and gluconic acid.

31. An aqueous disinfectant concentrate as claimed in claims 28 to 30, characterized in that it contains a formulation aid from the group consisting of ethanol, n-propanol, isopropanol, ethylene glycol and triacetin.

32. An aqueous disinfectant concentrate as claimed in claim 31, characterized in that it contains ethanol as formulation aid.

## Revendications

1. Procédé de nettoyage et de désinfection des endoscopes dans lequel :
a) on met en contact les surfaces à traiter des endoscopes avec une solution de nettoyage qui renferme au moins un agent tensioactif, non ionogène, peu moussant, au moins un enzyme protéolytique, au moins un agent complexant et, le cas échéant d'autres constituants de produits de nettoyage usuels,
b) on met en contact avec une solution d'agent désinfectant qui renferme au moins un aldéhyde choisi dans le groupe formé du formaldéhyde et des dialdéhydes aliphatiques ayant de 2 à 8 atomes de carbone et, au moins un agent complexant, et
c) on rince au moins deux fois avec de l'eau
caractérisé en ce que
- à l'étape a), la solution de nettoyage possède une valeur de pH allant de 6 à 8,
- on chauffe à 55 à 65°C,
- on maintient 1 à 15 minutes à cette température et ensuite on sépare,
- au stade b), la solution d'agent désinfectant possède une valeur de pH allant de 6 à 8,
- on chauffe à 55 à 65°C, et
- on maintient à cette température pendant 1 à 15 minutes,
- au stade c) on ajuste l'eau de rinçage à une valeur de pH allant de 6 à 8,
- dans lequel on chauffe l'eau au moins dans le dernier tour de rinçage à 55-65°C,
- on sèche dans un stade d), après le rinçage avec de l'air chaud stérilisé à 55-65°C, dans lequel on met en oeuvre aux étape a) à c) de l'eau ayant une dureté de 3 à 8° dH.

2. Procédé selon la revendication 1,
caractérisé en ce que
la solution de nettoyage au stade a) renferme :
0,1 à 1,0 g d'agent tensioactif peu moussant,
0,03 à 0,3 UA/l d'enzyme protéolytique et,
0,03 à 0,3 g d'agent complexant
(UA = Unités Anson).

3. Procédé selon les revendications 1 et 2,
caractérisé en ce que
la solution de nettoyage à l'étape a) renferme comme agent tensioactif non ionique et peu moussant, un produit d'addition d'oxyde d'alkylène qui est accessible par addition de 3 à 30 mol d'oxyde d'éthylène et/ou d'oxyde de propylène sur des polyols aliphatiques ayant de 2 à 6 groupes hydroxyle et de 2 à 12 atomes de carbone ainsi que sur des alcools gras, des acides gras, des amines grasses ou des alkylphénols ayant respectivement de 8 à 18 atomes de carbone, dans lesquels les groupes hydroxyle terminaux de ces dérivés d'éther de polyglycols peuvent être en outre éthérifiés, estérifiés ou acétalisés.

4. Procédé selon les revendications 1 à 3,
caractérisé en ce que
la solution de nettoyage à l'étape a) renferme un agent tensioactif non ionogène peu moussant choisi dans le groupe formé des produits d'addition de 3 à 15 mol d'oxyde d'éthylène sur des alcools gras saturés et non saturés ayant de 8 à 18 atomes de carbone, les produits d'addition de 3 à 5 mol d'oxyde d'éthylène et de 3 à 6 mot d'oxyde de propylène sur des alcools gras saturés et non saturés ayant de 8 à 18 atomes de carbone et des dérivés éther de ces éthers d'alcool gras et de polyalkylèneglycol dans lesquels les groupes hydroxyle terminaux sont éthérifiés avec un alcool aliphatique saturé à chaîne droite ou ramifié ayant de 4 à 8 atomes de carbone.

5. Procédé selon la revendication 4,
caractérisé en ce que
la solution de nettoyage à l'étape a) renferme comme agent tensioactif peu moussant un éther de polyéthylèneglycol de formule I,
R¹ - O - (CH₂CH₂O)ₙ - R² (I)
dans laquelle R¹ signifie un radical alkyle ou alkényle à chaîne droite ou ramifié, ayant de 8 à 18 atomes de carbone, R² signifie un radical alkyle à chaîne droite ou ramifiée ayant de 4 à 8 atomes de carbone, et n signifie un nombre de 7 à 12.

6. Procédé selon la revendication 5,
caractérisé en ce que
la solution de nettoyage à l'étape a) renferme un éther de polyéthylène glycol de formule I, à la revendication 5, dans laquelle R¹ représente un mélange qui dérive d'un alcools gras de suif, à base de radicaux alkyle et/ou alkényle ayant de 12 à 18 atomes de carbone et R² représente un radical n-butyle alors que n signifie un nombre allant de 9 à 10.

7. Procédé selon les revendications 1 à 6,
caractérisé en ce que
la solution de nettoyage à l'étape a) renferme un agent complexant choisi dans le groupe formé des sels alcalins de l'acide nitrilotriacétique, de l'acide éthylène diaminotétraacétique, de l'acide 1-hydroxyéthane-1,1-diphosphonique, de l'acide amino tris(méthylènephosphonique), de l' acide éthylène diaminotétrakis(méthylènephosphonique), de l'acide phosphonobutanetricarboxylique, de l'acide tartrique, de l'acide citrique et de l'acide gluconique.

8. Procédé selon la revendication 7,
caractérisé en ce que
la solution de nettoyage à l'étape a) contient comme agent complexant du gluconate de sodium.

9. Procédé selon les revendications 1 à 8,
caractérisé en ce que
la solution de produit désinfectant à l'étape b) contient : 0,5 à 5 g d'aldéhyde et 0,02 à 0,25 g/l d'agent complexant.

10. Procédé selon les revendications 1 à 9,
caractérisé en ce que
la solution de produit désinfectant à l'étape b) contient de l'aldéhyde glutarique.

11. Procédé selon les revendications 1 à 10,
caractérisé en ce que
la solution de produit désinfectant à l'étape b) contient un agent complexant choisi dans le groupe formé des sels alcalins de l'acide nitrilotriacétique, de l'acide éthylène diaminotétraacétique, de l'acide 1-hydroxyéthane-1,1-diphosphonique, de l'acide aminotris(méthylènephosphonique) de l'acide éthylène diaminotétrakis(méthylènephosphonique), de l'acide phosphonobutanetricarboxylique, de l'acide tartrique, de l'acide citrique et de l'acide gluconique.

12. Procédé selon la revendication 11,
caractérisé en ce que
la solution de produit désinfectant à l'étape b) contient comme agent complexant un sel de sodium de l'acide phosphonobutanetricarboxylique.

13. Procédé selon les revendications 1 à 12,
caractérisé en ce qu'
à l'étape a) la solution de nettoyage avant la séparation est additionnée d'une solution de produit désinfectant qui renferme au moins un aldéhyde choisi dans le groupe formé du formaldéhyde et des dialdéhydes ayant de 2 à 8 atomes de carbone et au moins un agent complexant.

14. Procédé selon la revendication 13,
caractérisé en ce que
la solution de nettoyage est déplacée avec autant de solution de produit désinfectant que dans la totalité de la solution, sont présents :
de 0,25 à 2,5 g/l d'aldéhyde et,
de 0,01 à 0,13 g/l d'agent complexant.

15. Procédé selon les revendications 1 à 14,
caractérisé en ce qu'
on rince les endoscopes que l'on doit traiter entre l'étape a) et l'étape b) au moins une fois avec de l'eau dont la valeur de pH est ajustée de 6 à 8.

16. Procédé selon les revendications 1 à 15,
caractérisé en ce qu'
on soumet à l'étape a) et/ou à l'étape b) les endoscopes à l'action des ultrasons.

17. Procédé selon les revendications 1 à 16,
caractérisé en ce qu'
on traite les endoscopes qui doivent être traités à l'étape d) avec de l'air chaud qui a été stérilisé à l'aide d'un microfiltre.

18. Concentré de produit de nettoyage aqueux pour l'exécution du procédé selon l'une des revendications 1 à 17 qui contient de 5 à 10 % en poids d'un agent non ionogène peu moussant, un enzyme protéolytique, un agent complexant, un agent stabilisant pour enzyme, un adjuvant de formulation et un agent de conservation,
caractérisé en ce que
l'agent tensioactif non ionogène peu moussant possède la formule :
R¹ - O - (CH₂CH₂O)ₙ - R² (I)
dans laquelle R¹ signifie un radical alkyle ou alkényle à chaîne droite ou ramifié, ayant de 8 à 18 atomes de carbone, R² signifie un radical alkyle à chaîne droite ou ramifiée ayant de 4 à 8 atomes de carbone, et n signifie un nombre de 7 à 12, la teneur en enzyme protéolytique dans la solution s'élève de 7,7 à 77 UA/l, la teneur en agent complexant s'élève de 1 à 5 % en poids, la teneur en agent stabilisant pour enzyme de 10 à 50 % en poids, la teneur en adjuvant de formulation de 1 à 5 % en poids et la teneur en agent de conservation de 0,05 à 0,5 % en poids et dont la valeur de pH est ajustée de 4 à 6.

19. Concentré aqueux de produit de nettoyage selon la revendication 18,
caractérisé en ce qu'
il renferme comme agent tensioactif un éther de polyéthylèneglycol de formule I dans laquelle R¹ représente un mélange provenant d'alcools gras de suif à base de radicaux alkyle et/ou alkénile, ayant de 8 à 18 atomes de carbone, et R² représente un radical n-butyle alors que n signifie un nombre allant de 9 à 10.

20. Concentré aqueux de produit de nettoyage selon les revendications 18 à 19,
caractérisé en ce qu'
il renferme un agent complexant choisi dans le groupe des sels alcalins de l'acide nitrilotriacétique, de l'acide éthylène diaminotétraacétique, de l'acide 1-hydroxyéthane 1,1-diphosphonique, de l'acide aminotris(méthylènephosphonique), de l'acide éthèlènediaminotétrakis(méthylènephosphonique), de l'acide phosphonobutane tricarboxylique, de l'acide tartrique, de l'acide citrique et de l'acide gluconique.

21. Concentré aqueux de produit de nettoyage selon la revendication 20,
caractérisé en ce qu'
il renferme, comme agent complexant, du gluconate de sodium.

22. Concentré aqueux de produit de nettoyage selon les revendications 18 à 20,
caractérisé en ce qu'
il renferme comme agent stabilisant pour enzyme, de la triéthanolamine, de la morpholine, de l'alpha-pyrrolidone, de l'éthylèneglycol, du propylèneglycol, du glycérol, des sels solubles dans l'eau de calcium ou des mélanges de ces composés.

23. Concentré aqueux de produit de nettoyage selon la revendication 22,
caractérisé en ce qu'
il renferme comme agent stabilisant pour enzyme du glycérol et/ou du propylèneglycol.

24. Concentré aqueux de produit de nettoyage selon les revendications 18 à 23,
caractérisé en ce qu'
il renferme comme adjuvant de formulation du cumène sulfonate de sodium, du toluène sulfonate de sodium, du xylène sulfonate de sodium, de l'urée, des polyéthylèneglycols des polypropylèneglycols, du méthylacétamide ou des alcools gras.

25. Concentré aqueux de produit de nettoyage selon la revendication 24,
caractérisé en ce qu'
il renferme comme adjuvant de formulation du cumène sulfonate de sodium.

26. Concentré aqueux de produit de nettoyage selon les revendications 18 à 25,
caractérisé en ce qu'
il renferme comme agent de conservation l'ester méthylique d'acide p-hydroxybenzoïque du 5-bromo 5-nitro 1,3-dioxane, de l'aldéhyde glutarique, de l'acide salicylique, du 0-2-natphtyl-m-N-diméthyl-thiocarbanilate, de la 5-chloror-5-méthyl-4-isothiazoline-3-one, de la 2-méthyl-4-isothiazoline-3-one ou un mélange de 5-chloro-5-méthyl-4-isothiazoline-3-one et de 2-méthyl-4-isothiaoline-3-one.

27. Concentré aqueux de produit de nettoyage selon la revendication 26,
caractérisé en ce qu'
il renferme comme agent de conservation de l'ester méthylique d'acide p-hydroxybenzoïque.

28. Concentré aqueux de produit désinfectant pour l'exécution du procédé selon l'une des revendications 1 à 17, renfermant un aldéhyde choisi dans le groupe formé du formaldéhyde et des dialdéhydes aliphatiques ayant de 2 à 8 atomes de carbone, un agent complexant et un adjuvant de formulation,
caractérisé en ce que
la teneur en cet aldéhyde s'élève de 10 à 40 % en poids, la teneur en agent complexant s'élève de 0,5 à 2 % en poids, et la teneur en adjuvant de formulation s'élève de 7 à 15 % en poids, dans lequel comme agent complexant de l'acide phosphonobutanetricarboxylique est contenu et dans lequel la valeur du pH du produit est ajustée de 3 à 5.

29. Concentré aqueux de produit désinfectant selon la revendication 28,
caractérisé en ce qu'
il renferme comme dialdéhyde aliphatique, de l'aldéhyde glutarique.

30. Concentré aqueux de produit désinfectant selon les revendications 28 et 29,
caractérisé en ce qu'
il renferme un agent complexant choisi dans le groupe des sels alcalins de l'acide nitrilotriacétique, de l'acide éthylène diaminotetraacétique, de l'acide 1-hydroxyéthane 1,1-diphosphonique, de l'acide aminotris(méthylène phosphonique) de l'acide éthylène diaminotétra tris(méthylènephosphonique), de l'acide phosphonobutanetricarboxylique, de l'acide tartrique, de l'acide citrique, et de l'acide gluconique.

31. Concentré aqueux de produit désinfectant selon les revendications 28 à 30,
caractérisé en ce qu'
il renferme un adjuvant de formulation choisi dans le groupe de l'éthanol, du n-propanol, de l'isopropanol, de l'éthylèneglycol et de la triacétine.

32. Concentré aqueux de produit désinfectant selon la revendication 31,
caractérisé en ce qu'
il renferme comme adjuvant de formulation, de l'éthanol.
